# EUROPEAN PATENT SPECIFICATION

(11) **EP 2 066 308 B1**
(45) Date of publication and mention of the grant of the patent: **21.07.2010**
(21) Application number: 07814963.0
(22) Date of filing: 21.09.2007
(51) Int. Cl.: A61K 9/40, A61K 9/42, A61K 9/44, A61K 9/20, A61K 9/26, A61K 9/28

(54) **MULTI-CORE SOLID DOSAGE FORM COMPRISING A TRANSPARENT COATING BETWEEN SAID CORES**
MEHRKERNIGE SOLIDE DOSIERFORM MIT EINER TRANSPARENTEN BESCHICHTUNG ZWISCHEN DEN KERNEN
FORME DE DOSAGE SOLIDE MULTI-CENTRE COMPRENANT UN REVÊTEMENT TRANSPARENT ENTRE LES CENTRES

(30) Priority: 25.09.2006 US 534845
(43) Date of publication of application: 10.06.2009
(73) Proprietor: Mcneil-PPC, Inc, Skillman, NJ 08558 (US)
(72) Inventor: LI, Shun, Por, Lansdale, Pennsylvania 19446 (US); NAEF, Hanspeter, Enola, Pennsylvania 17025 (US); BUNICK, Frank, Randolph, NJ 07869 (US); LEE, Der, Yang, Flemington, New Jersey 08822 (US)
(74) Representative: Kirsch, Susan Edith
(86) International application number: PCT/US2007/079148
(87) International publication number: WO 2008/039698

(56) References cited:
- EP-A- 1 602 363
- WO-A-00/18447
- WO-A-2004/028508

## Description

### FIELD OF THE INVENTION

This invention relates to dosage forms having two or more cores surrounded or covered at least in part by a shell that is translucent, preferably substantially transparent to the human eye. The cores are separate and distinct from one another such that along at least one axis of a major surface of at least one core a transparent coating is provided that is capable of transmitting light between said cores along the axis, preferably along at least two perpendicular axes drawn through the transparent coating and centered between the opposing cores.

### BACKGROUND OF THE INVENTION

Dosage forms have been previously designed with multiple cores housed in a single shell for the purpose of allowing flexibility in a dosing regimen. Published PCT application WO 00/18447, for example, describes a multiplex drug delivery system suitable for oral administration containing at least two distinct drug dosage packages, which exhibit equivalent dissolution profiles for an active agent when compared to one another and when compared to that of the entire multiplex drug delivery unit, and substantially enveloped by a scored compressed coating that allows the separation of the Multiplex drug delivery system into individual drug dosage packages.

U.S. Patent No. 6,113,945 relates to a multicolored medicament having a tablet core with a clear or single color uniform covering. Subsequent to the covering of the core, a coloring is provided over onto end of the core. This patent does not suggest a combination of compressed cores or the incorporation of an intermediate separating layer between such cores. Similarly, U.S. Patent 4,816,264 relates to a tablet having a core in which one or more coatings are provided over the core.

WO 2004/028508 describes a modified release dosage form comprising at least one active and at least two cores surrounded by a shell wherein the shell comprises at least one openings. EP 1602363 describes an immediate release dosage form having a solid core, a shell and one or more openings in the shell.

Improved dosage forms capable of differentiating from competing products and/or counterfeit products. This dosage form has a unique appearance and offers the benefit of being able to provide identifiers, markers or colorants into a translucent or light transmitting layer(s) of the dosage form. These identifiers, markers or colorants are in the form of colors and/or particles that are visible in full spectrum or when a specific wavelength light is transmitted through the at least translucent layer provided between compressed portions. The dosage forms comprise at least one active ingredient and at least two cores or compressed portions that surrounded or at least covered in party by a shell, wherein the shell is at least translucent, preferably substantially transparent.

### SUMMARY OF THE INVENTION

The invention provides a dosage form comprising at least one active ingredient, a first compressed portion, and a second compressed portion, said first and second compressed portions being surrounded by or least covered in party by a shell, wherein the shell is at least translucent, preferably substantially transparent such that at least some light can pass directly therethrough.

### BRIEF DESCRIPTION OF DRAWINGS

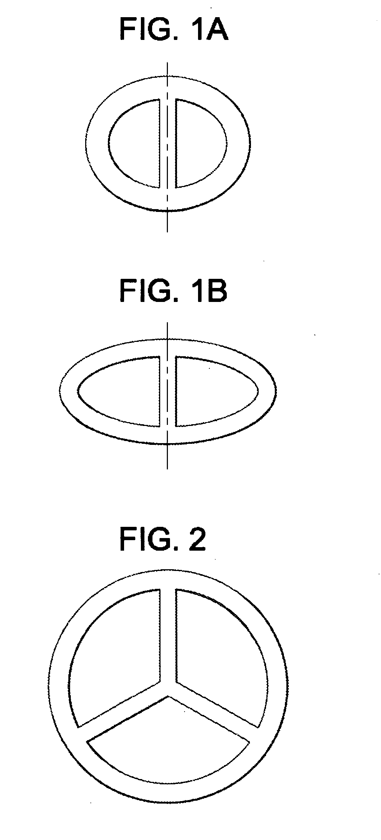

FIG. 1A depicts a cross-section of a dosage form according to the invention comprising first and seconds, side-by-side cores that are compressed tablets.
FIG. 1B shows a top view of the dosage form of FIG 1A.

### DETAILED DESCRIPTION OF THE INVENTION

As used herein, the term "dosage form" applies to any solid object, semi-solid, or liquid composition designed to contain a specific pre-determined amount (dose) of a certain ingredient, for example an active ingredient as defined below.

Suitable dosage forms may be pharmaceutical drug delivery systems, including those for oral administration, buccal administration, rectal administration, topical or mucosal delivery, or subcutaneous implants, or other implanted drug delivery systems; or compositions for delivering minerals, vitamins and other nutraceuticals, oral care agents, flavorants, and the like. Preferably the dosage forms of the present invention are considered to be solid, however they may contain liquid or semi-solid components. In a particularly preferred embodiment, the dosage form is an orally administered system for delivering a pharmaceutical active ingredient to the gastro-intestinal tract of a human.

Suitable active ingredients for use in this invention include for example pharmaceuticals, minerals, vitamins and other nutraceuticals, oral care agents, flavorants and mixtures thereof.

Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, oral contraceptives, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof.

Suitable oral care agents include breath fresheners, tooth whiteners, antimicrobial agents, tooth mineralizers, tooth decay inhibitors, topical anesthetics, mucoprotectants, and the like.

Suitable flavorants include menthol, peppermint, mint flavors, fruit flavors, chocolate, vanilla, bubblegum flavors, coffee flavors, liqueur flavors and combinations and the like.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotadine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrhcals, such as diphenoxylate and loperamide; glycopyrrolate; antiemetics, such as ondansctron, analgesics, such as mesalamine.

In one embodiment of the invention, the active ingredient may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the active ingredient is selected from analgesics, antiinflammatories, and antipyretics, e.g. non-steroidal anti-inflammatory drugs (NSAIDs), including propionic acid derivatives, e.g. ibuprofen, naproxen, ketoprofen and the like; acetic acid derivatives, e.g. indomethacin, diclofenac, sulindac, tolmetin, and the like; fenamic acid derivatives, e.g. mefanamic acid, meclofenamic acid, flufenamic acid, and the like; biphenylcarbodylic acid derivatives, e.g. diflunisal, flufenisal, and the like; and oxicams, e.g. piroxicam, sudoxicam, isoxicam, meloxicam, and the like. In one particular embodiment, the active ingredient is selected from propionic acid derivative NSAID, e.g. ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In another particular embodiment of the invention, the active ingredient may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another embodiment of the invention, the active ingredient may be selected from upper respiratory agents, such as pseudoephedrine, phenylpropanolamine, chlorpheniramine, dextromethorphan, diphenhydramine, astemizole, terfenadine, fexofenadine, loratadine, desloratadine, cetirizine, mixtures thereof and pharmaceutically acceptable salts, esters, isomers, and mixtures thereof.

The active ingredient or ingredients are present in the dosage form in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dosing regimen, the age and weight of the patient, and other factors must be considered, as known in the art. Typically, the dosage form comprises at least about 1 weight percent, for example, the dosage form comprises at least about 5 weight percent, say at least about 20 weight percent of a combination of one or more active ingredients. In one embodiment, a core comprises a total of at least about 25 weight percent (based on the weight of the core) of one or more active ingredients.

The active ingredient or ingredients may be present in the dosage form in any form. For example, the active ingredient may be dispersed at the molecular level, e.g. melted or dissolved, within the dosage form, or may be in the form of particles, which in turn may be coated or uncoated. If an active ingredient is in the form of particles , the particles (whether coated or uncoated) typically have an average particle size of about 1-2000 microns. In one embodiment, such particles are crystals having an average particle size of about 1-300 microns. In another embodiment, the particles are granules or pellets having an average particle size of about 50-2000 microns, for example about 50-1000 microns, say about 100-800 microns. In certain embodiments in which one or more active ingredients are in the form of particles, the active ingredient particles are contained within one or more cores of the dosage form.

Each core or compressed portion may be any solid form. As used herein, "core" or "compressed portion" refers to a part of the dosage form that is at least partially enveloped or surrounded by another material. Preferably, each core is a self-contained unitary object. Typically, a core comprises a solid, for example, a core may be a compressed or molded tablet, hard or soft capsule, suppository, or a confectionery form such as a lozenge, nougat, caramel, fondant, or fat based composition. In certain other embodiments, a core or a portion thereof may be in the form of a semi-solid or a liquid in the finished dosage form. For example a core may comprise a liquid filled capsule, or a semi-solid fondant material. In embodiments in which a core comprises a flowable component, such as a plurality of granules or particles, or a liquid, the core preferably additionally comprises an enveloping component, such as a capsule shell, or a coating, for containing the flowable material. In certain particular embodiments in which a core comprises an enveloping component, the shell or shell portions of the present invention are in direct contact with the enveloping component of the core, which separates the shell from the flowable component of the core.

The dosage form comprises at least two cores, e.g. a first core and a second core. The dosage form can comprise more than two cores. The cores can have the same or different compositions, comprise the same or different active ingredients, excipients (inactive ingredients that may be useful for conferring desired physical properties to the dosage core), and the like. One or more cores can be substantially free of active ingredient. The cores can even comprise incompatible ingredients from one another.

In one embodiment, each core is completely and separately surrounded by, or embedded in, a shell material. A portion of the shell, referred herein as the "interior wall" or "separating layer" separates the first and second cores. The distance between the first and second cores, i.e. thickness of the interior wall, may vary depending upon the desired release characteristics of the dosage form, or practical considerations related to the manufacturing process. For example, the thickness of the interior wall can be from about 10% to about 200% of the thickness of a core. In a particularly preferred embodiment, the separating layer is thick enough to allow light to be transmitted between two adjacent cores.

Each core may have one of a variety of different shapes. Each core may have the same or different physical dimensions, shape, etc. as the other cores. For example the first and second cores may have different diameters or thicknesses. For example, a core may be shaped as a polyhedron, such as a cube, pyramid, prism, or the like; or may have the geometry of a space figure with some non-flat faces, such as a cone, truncated cone, cylinder, sphere, torus, or the like. In certain embodiments, a core has one or more major faces. For example, in embodiments wherein a core is a compressed tablet, the core surface typically has opposing upper and lower faces formed by contact with the upper and lower punch faces in the compression machine. In such embodiments the core surface typically further comprises a "belly-band" located between the upper and lower faces, and formed by contact with the die walls in the compression machine. A core may also comprise a multilayer tablet.

In one embodiment at least one core is a compressed tablet having a hardness from about 2 to about 30 kp/cm², e.g. from about 6 to about 25 kp/cm² "Hardness" is a term used in the art to describe the diametral breaking strength of either the core or the coated solid dosage form as measured by conventional pharmaceutical hardness testing equipment, such as a Schleuniger Hardness Tester. In order to compare values across different size tablets, the breaking strength must be normalized for the area of the break. This normalized value, expressed in kp/cm², is sometimes referred in the art as tablet tensile strength. A general discussion of tablet hardness testing is found in Leiberman et al., Pharmaceutical Dosage Forms--Tablets, Volume 2, 2nd Ed., Marcel Dekker Inc., 1990, pp. 213-217, 327-329. In another embodiment, all the cores in the dosage form comprise a compressed tablet having a hardness from about 2 to about 30 kp/cm², e.g. from about 6 to about 25 kp/cm².

The first and second cores may be oriented side by side. For example, in the case of cores that are compressed tablets, their belly bands can be adjacent to and in contact with the interior wall. In certain examples the cores have top and bottom portions that have flat faces.

Alternatively, the cores may be oriented one on top of the other such that their upper or lower faces are adjacent to and in contact with the interior wall. The thickness of the shell may vary among various locations around the dosage form. For example, in embodiments where the cores have different sizes from one another, the shell may, as a result, have a smaller thickness around one core than the other. In embodiments where one or more cores have a different shape than that of the surrounding shell surface, the shell thickness will be different around certain portions of a core than around certain other portions. In embodiments where the shell comprises more than one portion, the shell portions may have different thickness from one another at corresponding locations. In embodiments where the cores are positioned asymmetrically within the dosage form, the shell thickness will vary accordingly. This may be exploited to adjust the relative onset or rate of release of active ingredient from the two cores. For example, active ingredient contained in a smaller core could be released after the release of active ingredient from a larger core has begun, due to the relative thinness of the shell around the larger core. In another example, active ingredient contained in a first, elongated, core could begin to be released sooner than active ingredient from a second, more symmetrically shaped core due to the relative thinness of the shell proximal to the elongated portion of the first core.

In another embodiment the dosage form has three cores equally separated from one another. In a particular version of this embodiment the belly bands of the three portions have circular outside edges with approximately 135° angles at the interior edges, adjacent to each opposing core. This dosage form allows for light to be transmitted through the top and bottom of the dosage form, while blocking light that is transmitted through the side of the dosage form.

In another embodiment the shell on the top portion of the dosage form comprises one color and the shell on the bottom portion of the dosage form comprises a second color. When light is transmitted through this embodiment of the dosage form it displays a unique color, e.g. blue on top and yellow on bottom display a green composite color.

In another embodiment the shell portions comprise particles or sparkled flakes that display separate effects or diffract or reflect light at different angles or under light. In embodiments where particle or flakes are added to the shell, the particles or flakes are made of materials such as but are not limited to titanium dioxide, aluminum lakes, magnesium lakes, calcium takes, mica, pearlescent colors, fluorescent materials, and flavorants.

In one embodiment, the shell portions optionally comprise a flavoring agent or sensate. As used herein, a "sensate" is a chemical agent that elicits a sensory effect in the mouth, nose, and/or throat other than aroma or flavor. Examples of such sensory effects include, but are not limited to, cooling, warming, tingling, mouth watering (succulent), astringent, and the like. Sensate agents suitable for use in the present invention are commercially available and may be purchased from, for example, International Flavor & Fragrances. In one embodiment the shell portions of the dosage form comprise a light transmitting layer, which comprise a film forming material and a material, which absorbs light in wavelengths outside of the visible spectrum (i.e. ultraviolet) light. This can be achieved by adding an ultraviolet dye to the shell material. This provides for unique identification of dosage form when an ultraviolet source is shined on the dosage form.

In certain embodiments the dosage form allows for 50% light transmission through the dosage form, as evidenced by the light transmitted through the translucent shell portions, or about more than 10% light transmission, or about more than 5% light transmission or about more than 1% light transmission or about more 0.05%. In embodiment where the dosage form allows for 10% of light to be transmitted through the dosage form, is equivalent to 90% of light being diffracted.

In certain embodiments the thickness of the light transmitting layer between the compressed dosage form core portions is about 0.1 to about 2 mm, or about 0.25 to about 1.5 mm or about 0.5 to about 1.25 mm..

Exemplary core shapes that may be employed include tablet shapes formed from compression tooling shapes described by "The Elizabeth Companies Tablet Design Training Manual" (Elizabeth Carbide Die Co., Inc., p. 7 (McKeesport, Pa.) (incorporated herein by reference) as follows (the tablet shape corresponds inversely to the shape of the compression tooling):
1. Shallow Concave. 2. Standard Concave. 3. Deep Concave. 4. Extra Deep Concave. 5. Modified Ball Concave. 6. Standard Concave Bisect. 7. Standard Concave Double Bisect. 8. Standard Concave European Bisect. 9. Standard Concave Partial Bisect. 10. Double Radius. 11. Bevel & Concave. 12. Flat Plain. 13. Flat-Faced-Beveled Edge (F.F.B.E.). 14. F.F.B.E. Bisect. 15. F.F.B.E. Double Bisect. 16. Ring. 17. Dimple. 18. Ellipse. 19. Oval. 20. Capsule. 21. Rectangle. 22. Square. 23. Triangle. 24. Hexagon. 25. Pentagon. 26. Octagon. 27. Diamond. 28. Arrowhead. 29. Bullet. 30. Shallow Concave. 31. Standard Concave. 32. Deep Concave. 33. Extra Deep Concave. 34. Modified Ball Concave. 35. Standard Concave Bisect. 36. Standard Concave Double Bisect. 37. Standard Concave European Bisect. 38. Standard Concave Partial Bisect. 39. Double Radius. 40. Bevel & Concave. 41. Flat Plain. 42. Flat-Faced-Beveled Edge (F.F.B.E.). 43. F.F.B.E. Bisect. 44. F.F.B.E. Double Bisect. 45. Ring. 46. Dimple. 47. Ellipse. 48. Oval. 49. Capsule. 50. Rectangle. 51. Square. 52. Triangle. 53. Hexagon. 54. Pentagon. 55. Octagon. 56. Diamond. 57. Arrowhead. 58. Bullet. 59. Barrel. 60. Half Moon. 61. Shield. 62. Heart. 63. Almond. 64. House/Home Plate. 65. Parallelogram. 66. Trapezoid. 67. Bar Bell. 68. Bow Tic. 69. Uneven Triangle.

The cores may be prepared by any suitable method, including for example compression or molding, and depending on the method by which they are made, typically comprise active ingredient and a variety of excipients. The cores may be prepared by the same or different methods. For example, a first core may be prepared by compression, and a second core may be prepared by molding, or both cores may be prepared by compression. In embodiments in which one or more cores, or portions thereof are made by compression, suitable excipients include fillers, binders, disintegrants, lubricants, glidants, and the like, as known in the art. In embodiments in which a core is made by compression and additionally confers modified release of an active ingredient contained therein, such core preferably further comprises a release-modifying compressible excipient.

Suitable fillers for use in making a core or core portion by compression include water-soluble compressible carbohydrates such as sugars, which include dextrose, sucrose, maltose, and lactose, starches, corn starch, sugar-alcohols, which include mannitol, sorbitol, maltitol, xylitol, starch hydrolysates, which include dextrins, and maltodextrins, and the like, water insoluble plastically deforming materials such as microcrystalline cellulose or other cellulosic derivatives, water-insoluble brittle fracture materials such as dicalcium phosphate, tricalcium phosphate and the like and mixtures thereof.

Suitable binders for making a core or core portion by compression include dry binders such as polyvinyl pyrrolidone, hydroxypropylmethylcellulose, and the like; wet binders such as water-soluble polymers, including hydrocolloids such as acacia, alginates, agar, guar gum, locust bean, carrageenan, carboxymethylcellulose, tara, gum arabic, tragacanth, pectin, xanthan, gellan, gelatin, maltodextrin, galactomannan, pusstulan, laminarin, scleroglucan, inulin, whelan, rhamsan, zooglan, methylan, chitin, cyclodextrin, chitosan, polyvinyl pyrrolidone, cellulosics, sucrose, starches, and the like; and derivatives and mixtures thereof.

Suitable disintegrants for making a core or core portion by compression, include sodium starch glycolate, cross-linked polyvinylpyrrolidone, cross-linked carboxymethylcellulose, starches, microcrystalline cellulose, and the like.

Suitable lubricants for making a core or core portion by compression include long chain fatty acids and their salts, such as magnesium stearate and stearic acid, talc, glycerides and waxes.

Suitable glidants for making a core or core portion by compression, include colloidal silicon dioxide, and the like.

Suitable release-modifying excipients for making a core or core portion by compression include swellable erodible hydrophilic materials, insoluble edible materials, pH-dependent polymers, and the like.

Suitable swellable erodible hydrophilic materials for use as release-modifying excipients for making a core or core portion by compression include: water swellable cellulose derivatives, polyalkylene glycols, thermoplastic polyalkylene oxides, acrylic polymers, hydrocolloids, clays, gelling starches, and swelling cross-linked polymers, and derivatives, copolymers, and combinations thereof. Examples of suitable water swellable cellulose derivatives include sodium carboxymethylcellulose, cross-linked hydroxypropylcellulose, hydroxypropyl cellulose (HPC), hydroxypropylmethylcellulose (HPMC), hydroxylsopropylcellulose, hydroxybutylcellulose, hydroxyphenylcellulose, hydroxyethylcellulose (HEC), hydroxypentylcellulose, hydroxypropylethylcellulose, hydroxypropylbutylcellulose, hydroxypropylethylcellulose. Examples of suitable polyalkylene glycols include polyethylene glycol. Examples of suitable thermoplastic polyalkylene oxides include poly (ethylene oxide). Examples of suitable acrylic polymers include potassium methacrylatedivinylbenzene copolymer, polymethylmethacrylate, CARBOPOL (high-molecular weight cross-linked acrylic acid homopolymers and copolymers), and the like. Examples of suitable hydrocolloids include alginates, agar, guar gum, locust bean gum, kappa carrageenan, iota carrageenan, tara, gum arabic, tragacanth, pectin, xanthan gum, gellan gum, maltodextrin, galactomannan, pusstulan, laminarin, scleroglucan, gum arabic, inulin, pectin, gelatin, whelan, rhamsan, zooglan, methylan, chitin, cyclodextrin, chitosan. Examples of suitable clays include smectites such as bentonite, kaolin, and laponite; magnesium trisilicate, magnesium aluminum silicate, and the like, and derivatives and mixtures thereof. Examples of suitable gelling starches include acid hydrolyzed starches, swelling starches such as sodium starch glycolate, and derivatives thereof. Examples of suitable swelling cross-linked polymers include cross-linked polyvinyl pyrrolidone, cross-linked agar, and cross-linked carboxymethylcellose sodium.

Suitable insoluble edible materials for use as release-modifying excipients for making a core or core portion by compression include water-insoluble polymers, and low-melting hydrophobic materials. Examples of suitable water-insoluble polymers include ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof.

Suitable low-melting hydrophobic materials include fats, fatty acid esters, phospholipids, and waxes. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes include carnauba wax, spermaceti wax, beeswax, candelilla wax, shellac wax, microcrystalline wax, and paraffin wax; fat-containing mixtures such as chocolate; and the like.

Suitable pH-dependent polymers for use as release-modifying excipients for making a core or core portion by compression include enteric cellulose derivatives, for example hydroxypropyl methylcellulose phthalate, hydroxypropyl methylcellulose acetate succinate, cellulose acetate phthalate; natural resins such as shellac and zein; enteric acetate derivatives such as for example polyvinylacetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename EUDRAGIT S, and poly(methacrylic acid, methyl methacrylate) 1:1, which is commercially available from Rohm Pharma GmbH under the tradename EUDRAGIT L, and the like, and derivatives, salts, copolymers, and combinations thereof.

Suitable pharmaceutically acceptable adjuvants for making a core or core portion by compression include, preservatives; high intensity sweeteners such as aspartame, acesulfame potassium, sucralose, and saccharin; flavorants; colorants; antioxidants; surfactants; wetting agents; and the like and mixtures thereof.

In embodiments wherein one or more cores are prepared by compression, a dry blending (i.e. direct compression), or wet granulation process may be employed, as known in the art. In a dry blending (direct compression) method, the active ingredient or ingredients, together with the excipients, are blended in a suitable blender, than transferred directly to a compression machine for pressing into tablets. In a wet granulation method, the active ingredient or ingredients, appropriate excipients, and a solution or dispersion of a wet binder (e.g. an aqueous cooked starch paste, or solution of polyvinyl pyrrolidone) are mixed and granulated. Alternatively a dry binder may be included among the excipients, and the mixture may be granulated with water or other suitable solvent.

Suitable apparatuses for wet granulation are known in the art, including low shear, e.g. planetary mixers; high shear mixers; and fluid beds, including rotary fluid beds. The resulting granulated material is dried, and optionally dry-blended with further ingredients, e.g. adjuvants and/or excipients such as for example lubricants, colorants, and the like. The final dry blend is then suitable for compression. Methods for direct compression and wet granulation processes are known in the art, and are described in detail in, for example, Lachman, et al., The Theory and Practice of Industrial Pharmacy, Chapter 11 (3rd ed. 1986).

The dry-blended, or wet granulated, powder mixture is typically compacted into tablets using a rotary compression machine as known in the art, such as for example those commercially available from Fette America Inc., Rockaway, N.J., or Manesty Machines LTD, Liverpool, UK. In a rotary compression machine, a metered volume of powder is filled into a die cavity, which rotates as part of a "die table" from the filling position to a compaction position where the powder is compacted between an upper and a lower punch to an ejection position where the resulting tablet is pushed from the die cavity by the lower punch and guided to an ejection chute by a stationary "take-off" bar.

In one embodiment, at least one core is prepared by the compression methods and apparatus described in issued U.S. Patent No. 6,767,200, the disclosure of which is incorporated herein by reference. Specifically, the core is made using a rotary compression module comprising a fill zone, compression zone, and ejection zone in a single apparatus having a double row die construction as shown in FIG.6 therein. The dies of the compression module are preferably filled using the assistance of a vacuum, with filters located in or near each die.

Cores made by compression may be single or multi-layer, for example bi-layer, tablets. A shell material surrounds or coats at least part of each of the cores. There must be at least enough shell material in contact with each of the cores to provide for at least one intermediately positioned translucent layer between two cores. The shell, in one embodiment, is continuous and completely surrounds both of the cores. In an alternative embodiment, the cores may be tapered or shaped such that a small part of one surface are in such close proximity or touching that shell material is unable to completely surround both cores. However, a major portion of the adjacent, facing surfaces are spaced sufficiently to allow shell material to be provided in the gap or space between at least two cores.

The shell can be a single, unitary coating, or the shell can comprise multiple portions, e.g. a first shell portion and a second shell portion. In certain embodiments the shell or shell portions are in direct contact with one or more cores or core portion. In certain other embodiments, the shell or shell portions are in direct contact with a subcoating or enveloping component that substantially surrounds a core or core portion. In embodiments, in which multiple shell portions are employed, the shell portions can have the same or different compositions and shapes from one another.

In certain embodiments the dosage form comprises a first shell portion and a second shell portion that are compositionally different. As used herein, the term "compositionally different" means having features that are readily distinguishable by qualitative or quantitative chemical analysis, physical testing, or visual observation. For example, the first and second shell portions may contain different ingredients, or different levels of the same ingredients, or the first and second shell portions may have different physical or chemical properties, different functional properties, or be visually distinct. Examples of physical or chemical properties that may be different include hydrophylicity, hydrophobicity, hygroscopicity, elasticity, plasticity, tensile strength, crystallinity, and density. Examples of functional properties which may be different include rate and/or extent of dissolution of the material itself or of an active ingredient therefrom, rate of disintegration of the material, permeability to active ingredients, permeability to water or aqueous media, and the like. Examples of visual distinctions include size, shape, topography, or other geometric features, color, hue, opacity, and gloss.

In one embodiment, the first core is surrounded by a first shell portion, and the second core is surrounded by a second shell portion. For example, in one particular such embodiment, the first and second cores may contain the same active ingredient in the same amount, and may be essentially identical in size, shape, and composition, while the first and second shell portions are have different dissolution properties, and confer different release profiles to the active ingredient portions contained in the first and second cores.

In another embodiment, the first and second cores are oriented side by side, for example as two compressed tablets with their belly bands adjacent to and in contact with the interior wall. The upper faces of both cores may be in contact with a first shell portion, and the lower faces of both cores may be in contact with a second shell portion. In certain other embodiments in which the first and second cores are compressed or molded tablets oriented one on top of the other such that their upper or lower faces are adjacent to and in contact with the interior wall, one core may be entirely surrounded by a first shell portion, and the other core may be entirely surrounded by a second shell portion.

In another embodiment, one or more portions of the first and/or second cores are not covered by any shell material. The uncovered portions can be small apertures or openings that expose the core to the liquid medium in which the dosage form is delivered or could expose some or all of one or more major surfaces on the core(s). In one embodiment, all of the surfaces of the core other than the surface adjacent to the opposing core are uncovered. In a still further embodiment, three cores are provided horizontally in a manner in which all of the surfaces other than the surfaces facing another core are exposed while shell material is provided between each of the cores.

In one embodiment, the surface of the first or second core is substantially totally coated with a subcoating. In this embodiment, a shell comprising first and second shell portions is in direct contact with the surface of the subcoating. As used herein, "substantially totally covering" means at least about 95 percent of the surface area of the core is covered by the subcoating.

The use of subcoatings is well known in the art and disclosed in, for example, U.S. Pat. Nos. 3,185,626, which is incorporated by reference herein. Any composition suitable for film-coating a tablet may be used as a subcoating according to the present invention. Examples of suitable subcoatings are disclosed in U.S. Pat. Nos. 4,683,256, 4,543,370, 4,643,894, 4,828,841, 4,725,441, 4,802,924, 5,630,871, and 6,274,162, which are all incorporated by reference herein. Additional suitable subcoatings include one or more of the following ingredients: cellulose ethers such as hydroxypropylmethylcellulose, hydroxypropylcellulose, and hydroxyethylcellulose; polycarbohydrates such as xanthan gum, starch, and maltodextrin; plasticizers including for example, glycerin, polyethylene glycol, propylene glycol, dibutyl sebecate, triethyl citrate, vegetable oils such as castor oil, surfactants such as Polysorbate-80, sodium lauryl sulfate and dioctyl-sodium sulfosuccinate; polycarbohydrates, pigments, and opacifiers.

In one embodiment, the subcoating comprises, based upon the total weight of the subcoating, from about 2 percent to about 8 percent, e.g. from about 4 percent to about 6 percent of a water-soluble cellulose ether and from about 0.1 percent to about 1 percent, castor oil, as disclosed in detail in U.S. Pat. No. 5,658,589, which is incorporated by reference herein. In another embodiment, the subcoating comprises, based upon the total weight of the subcoating, from about 20 percent to about 50 percent, e.g., from about 25 percent to about 40 percent of HPMC; from about 45 percent to about 75 percent, e.g., from about 50 percent to about 70 percent of maltodextrin; and from about I percent to about 10 percent, e.g., from about 5 percent to about 10 percent of PEG 400.

In embodiments in which a subcoating is employed, the dried subcoating typically is present in an amount, based upon the dry weight of the core, from about 0 percent to about 5 percent.

In another embodiment, one or more cores, e.g. all the cores, are substantially free of subcoating, and the shell or a shell portion is in direct contact with a core surface.

FIG. 1A depicts a cross-sectional top view of a dosage form according to the invention comprising first and second, side-by-side cores that are compressed. A shell of light transmitting material is provided over, around and between the cores. FIG. 1B shows a cross-section side view of the dosage form of FIG. 1A. The axis shown in FIG. 1A is considered, for purposes of this application, a major axis because it is longer than the axis through the light-transmitting layer of FIG. 1B. FIG. 2 is a representation of a three core configuration with a shell of light transmitting material.

The active ingredient or ingredients may be found within one or more cores, the shell, or portions or combinations thereof. Preferably, one or more active ingredients are contained in one or more cores. More preferably, at least one active ingredient is contained in each of the first and second cores.

The shell material, in its final form, is at least translucent. Translucent materials transmit light but cause diffusion to an extent that objects therein and beyond can not be seen clearly. Transparent materials, on the other hand, allow light to pass without appreciable light scattering so that objects lying therein and beyond can be clearly seen.

In certain embodiments the shell material have in its final form sufficient light transmissibility can be made from suitable gelling film forming materials such as gelatin, locust bean gum, gellan gum, carageenan and starch. In other embodiments the shell material have in its final form water soluble film forming materials such as hypromellose, polyvinyl alcohol, polyethylene glycol, hydroxypropylcellulose, and methylcellulose. The certain embodiments the shell contains a colorant in the form of a lake or a water soluble dye.

In certain embodiments the shell comprises, based upon the total weight of the shell, from about 1 percent to about 50 percent, e.g., from about 1 percent to about 30 percent of a plasticizer. Examples of suitable plasticizers include, but are not limited to polyethylene glycol; propylene glycol; glycerin; sorbitol; triethyl citrate; tributyl citrate; dibutyl sebecate; vegetable oils such as castor oil, rape oil, olive oil, and sesame oil; surfactants such as polysorbates, sodium lauryl sulfates, and dioctyl-sodium sulfosuccinates; mono acetate of glycerol; diacetate of glycerol; triacetate of glycerol; natural gums; triacetin; acetyltributyl citrate; diethyloxalate; diethylmalate; diethyl fumarate; diethylmalonate; dioctylphthalate; dibutylsuccinate; glyceroltributyrate; hydrogenated castor oil; fatty acids; substituted triglycerides and glycerides; and the like and/or mixtures thereof.

The composition of the shell can function to modify the release therethrough of an active ingredient contained in an underlying core. In one embodiment, the shell may function to delay release of an active ingredient from an underlying core. In another embodiment, the shell may function to sustain, extend, retard, or prolong the release of at least one active ingredient from the second (distally located) core.

Suitable swellable erodible hydrophilic materials for use as release modifying moldable excipients include water swellable cellulose derivatives, polyalkylene glycols, thermoplastic polyalkylene oxides, acrylic polymers, hydrocolloids, clays, gelling starches, and swelling cross-linked polymers, and derivatives, copolymers, and combinations thereof. Examples of suitable water swellable cellulose derivatives include sodium carboxymethylcellulose, cross-linked hydroxypropylcellulose, hydroxypropyl cellulose (HPC), hydroxypropylmethylcellulose (HPMC), hydroxyisopropylcellulose, hydroxybutylcellulose,hydroxyphenylcellulose, hydroxyethylcellulose (HEC), hydroxypentylcellulose, hydroxypropylethylcellulose, hydroxypropylbutylcellulose, hydroxypropylethylcellulose. Examples of suitable polyalkylene glycols include polyethylene glycol. Examples of suitable thermoplastic polyalkylene oxides include poly (ethylene oxide). Examples of suitable acrylic polymers include potassium methacrylatedivinylbenzene copolymer, polymethylmethacrylate, CARBOPOL (high-molecular weight cross-linked acrylic acid homopolymers and copolymers), and the like. Examples of suitable hydrocolloids include alginates, agar, guar gum, locust bean gum, kappa carrageenan, iota carrageenan, tara, gum arabic, tragacanth, pectin, xanthan gum, gellan gum, maltodextrin, galactomannan, pusstulan, laminarin, scleroglucan, gum arabic, inulin, pectin, gelatin, whelan, rhamsan, zooglan, methylan, chitin, cyclodextrin, chitosan. Examples of suitable clays include smectites such as bentonite, kaolin, and laponite; magnesium trisilicate, magnesium aluminum silicate, and the like, and derivatives and mixtures thereof. Examples of suitable gelling starches include acid hydrolyzed starches, swelling starches such as sodium starch glycolate, and derivatives thereof. Examples of suitable swelling cross-linked polymers include cross-linked polyvinyl pyrrolidone, cross-linked agar, and cross-linked carboxymethylcellose sodium.

Suitable pH-dependent polymers for use as release-modifying moldable excipients include enteric cellulose derivatives, for example hydroxypropyl methylcellulose phthalate, hydroxypropyl methylcellulose acetate succinate, cellulose acetate phthalate; natural resins such as shellac and zein; enteric acetate derivatives such as for example polyvinylacetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename EUDRAGIT S, and poly(methacrylic acid, methyl methacrylate) 1:1, which is commercially available from Rohm Pharma GmbH under the tradename EUDRAGIT L, and the like, and derivatives, salts, copolymers, and combinations thereof.

Suitable insoluble edible materials for use as release-modifying moldable excipients include water-insoluble polymers, and low-melting hydrophobic materials. Examples of suitable water-insoluble polymers include ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof.

Suitable low-melting hydrophobic materials include fats, fatty acid esters, phospholipids, and waxes. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes include carnauba wax, spermaceti wax, beeswax, candelilla wax, shellac wax, microcrystalline wax, and paraffin wax; fat-containing mixtures such as chocolate; and the like.

Accordingly, in certain embodiments, the dosage form comprises at least two cores containing the same or different active ingredient surrounded by a shell comprising a release modifying moldable excipient. In certain embodiments, the shell itself, e.g. a portion thereof, or an outer coating thereon may also contain colorant, such as a dye or pigment. In one embodiment, the colorant is visible. In another embodiment, the colorant is visible only when full spectrum or specific wavelength light is cause to be transmitted through the shell material. In another embodiment, the shell material is provided with reflective materials that reflect and diffract a portion of light transmitted therethrough. In certain preferred embodiments of the invention, the cores, the shell, any portions thereof, or both are prepared by molding. In particular, the cores, the shell, or both may be made by solvent-based molding or solvent-free molding. In such embodiments, the core or the shell is made from a flowable material optionally comprising active ingredient.

The flowable material can be an edible material that is flowable at a temperature between about 37°C and 250°C, and that is solid, semi-solid, or can form a gel at a temperature between about -10°C and about 35°C. When it is in the fluid or flowable state, the flowable material may comprise a dissolved or molten component for solvent-free molding, or optionally a solvent such as, for example, water or organic solvents, or combinations thereof, for solvent-based molding. The solvent may be partially or substantially removed by drying.

In one embodiment, solvent-based or solvent-free molding is performed via thermal setting molding using the method and apparatus described in issued U.S. Patent No. 6,892,094, the disclosure of which is incorporated herein by reference. In this embodiment, a core or shell is formed by injecting flowable form into a molding chamber. The flowable material preferably comprises a thermal setting material at a temperature above its melting point but below the decomposition temperature of any active ingredient contained therein. The flowable material is cooled and solidifies in the molding chamber into a shaped form (i.e., having the shape of the mold).

According to this method, the flowable material may comprise solid particles suspended in a molten matrix, for example a polymer matrix. The flowable material may be completely molten or in the form of a paste. The flowable material may comprise an active ingredient dissolved in a molten material in the case of solvent-free molding. Alternatively, the flowable material may be made by dissolving a solid in a solvent, which solvent is then evaporated after the molding step in the case of solvent-based molding.

The mold units may comprise center mold assemblies 212, upper mold assemblies 214, and lower mold assemblies 210, as shown in FIGS. 26-28, which mate to form mold cavities having a desired shape, for instance of a core or a shell surrounding one or more cores. As rotor 202 rotates, opposing center and upper mold assemblies or opposing center and lower mold assemblies close. Flowable material, which is heated to a flowable state in reservoir 206, is injected into the resulting mold cavities. The temperature of the flowable material is then decreased, hardening the flowable material. The mold assemblies open and eject the finished product.

In a particularly preferred embodiment of the invention, the shell is applied to the dosage form using a thermal cycle molding apparatus of the general type shown in FIGS. 28A-C of copending U.S. published application 20030086973 comprising rotatable center mold assemblies 212, lower mold assemblies 210 and upper mold assemblies 214. Cores are continuously fed to the mold assemblies. Shell flowable material, which is heated to a flowable state in reservoir 206, is injected into the mold cavities created by the closed mold assemblies holding the cores. The temperature of the shell flowable material is then decreased, hardening it around the cores. The mold assemblies open and eject the finished dosage forms. Shell coating is performed in two steps, each half of the dosage forms being coated separately via rotation of the center mold assembly.
In particular, the mold assemblies for applying the shell are provided with two or more cavities to accommodate the desired number of cores in the dosage form. A wall, preferably made of rubber or metal, separates the cavities and the overall shape of the cavities conform to the shape of the cores. One or more mold cavities can be provided with protrusions or masking elements to provide apertures, shapes, texture, or openings as desired in the shell material and/or control the surface area covered by the shell material.

In one embodiment, the compression module of U.S. Patent No. 6,767,200 may be employed to make cores. The shell may be made applied to these cores using a molding module as described above. A transfer device may be used to transfer the cores from the compression module to the molding module. Each transfer unit comprises multiple retainers for holding multiple cores side by side. In one embodiment, the retainers within each transfer unit are adjusted via a cam track/cam follower mechanism as the transfer units move around the transfer device. On arrival at the molding module, the cores grouped together for placement in a single dosage form, which have been held within a single transfer unit, are properly spaced from one another and ready to be fed into the mold assemblies. The cores may or may not have the same composition, as desired. The cores may comprise a single layer or multiple layers.

Alternatively, if cores of the same composition are to be used in the dosage forms, the compression module may be equipped with multi-tip compression tooling. Four-tip tooling, for example, may be used to make four cores within one die. The cores may comprise a single layer of multiple layers.

Suitable thermoplastic materials for use in or as the flowable material include both water-soluble and water insoluble polymers that are generally linear, not crosslinked, and not strongly hydrogen bonded to adjacent polymer chains. Examples of suitable thermoplastic materials include: thermoplastic water swellable cellulose derivatives, thermoplastic water insoluble cellulose derivatives, thermoplastic vinyl polymers, thermoplastic starches, thermoplastic polyalkylene glycols, thermoplastic polyalkylene oxides, and amorphous sugar-glass, and the like, and derivatives, copolymers, and combinations thereof. Examples of suitable thermoplastic water swellable cellulose derivatives include hydroxypropyl cellulose (HPC), hydroxypropylmethyl cellulose (HPMC), methyl cellulose (MC). Examples of suitable thermoplastic water insoluble cellulose derivatives include cellulose acetate (CA), ethyl cellulose (EC), cellulose acetate butyrate (CAB), and cellulose propionate. Examples of suitable thermoplastic vinyl polymers include polyvinyl alcohol (PVA) and polyvinyl pyrrolidone (PVP). Examples of suitable thermoplastic starches are disclosed for example in U.S. Pat. No. 5,427,614.

Examples of suitable thermoplastic polyalkylene glycols include polyethylene glycol. Examples of suitable thermoplastic polyalkylene oxides include polyethylene oxide having a molecular weight from about 100,000 to about 900,000 Daltons. Other suitable thermoplastic materials include sugar in the form on an amorphous glass such as that used to make hard candy forms.

Any film former known in the art is suitable for use in the flowable material. Examples of suitable film formers include, but are not limited to, film-forming water soluble polymers, film-forming proteins, film-forming water insoluble polymers, and film-forming pH-dependent polymers. In one embodiment, the film-former for making the core or shell or portion thereof by molding may be selected from cellulose acetate, ammonio methacrylate copolymer type B, shellac, hydroxypropylmethylcellulose, and polyethylene oxide, and combinations thereof.

Suitable film-forming water soluble polymers include water soluble vinyl polymers such as polyvinylalcohol (PVA); water soluble polycarbohydrates such as hydroxypropyl starch, hydroxyethyl starch, pullulan, methylethyl starch, carboxymethyl starch, pre-gelatinized starches, and film-forming modified starches; water swellable cellulose derivatives such as hydroxypropyl cellulose (HPC), hydroxypropylmethyl cellulose (HPMC), methyl cellulose (MC), hydroxyethylmethylcellulose (HEMC), hydroxybutylmethylcellulose (HBMC), hydroxyethylethylcellulose (HEEC), and hydroxyethylhydroxypropylmethyl cellulose (HEMPMC); water soluble copolymers such as methacrylic acid and methacrylate ester copolymers, polyvinyl alcohol and polyethylene glycol copolymers, polyethylene oxide and polyvinylpyrrolidone copolymers; and derivatives and combinations thereof.

Suitable film-forming proteins may be natural or chemically modified, and include gelatin, whey protein, myofibrillar proteins, coagulatable proteins such as albumin, casein, caseinates and casein isolates, soy protein and soy protein isolates, zein; and polymers, derivatives and mixtures thereof.

Suitable film-forming water insoluble polymers, include for example ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof.

Suitable film-forming pH-dependent polymers include enteric cellulose derivatives, such as for example hydroxypropyl methylcellulose phthalate, hydroxypropyl methylcellulose acetate succinate, cellulose acetate phthalate; natural resins, such as shellac and zein; enteric acetate derivatives such as for example polyvinylacetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename, EUDRAGIT S, and poly(methacrylic acid, methyl methacrylate) 1:1, which is commercially available from Rohm Pharma GmbH under the tradename, EUDRAGIT L, and the like, and derivatives, salts, copolymers, and combinations thereof.

One suitable hydroxypropylmethylcellulose compound for use as a thermoplastic film-forming water soluble polymer is "HPMC 291 ", which is a cellulose ether having a degree of substitution of about 1.9 and a hydroxypropyl molar substitution of 0.23, and containing, based upon the total weight of the compound, from about 29% to about 30% methoxyl groups and from about 7% to about 12% hydroxylpropyl groups. HPMC 2910 is commercially available from the Dow Chemical Company under the tradename METHOCEL E. METHOCEL E5, which is one grade of HPMC-2910 suitable for use in the present invention, has a viscosity of about 4 to 6 cps (4 to 6 millipascal-seconds) at 20C in a 2% aqueous solution as determined by a Ubbelohde viscometer. Similarly, METHOCEL E6, which is another grade of HPMC-2910 suitable for use in the present invention, has a viscosity of about 5 to 7 cps (5 to 7 millipascal-seconds) at 20C in a 2% aqueous solution as determined by a Ubbelohde viscometer. METHOCEL E15, which is another grade of HPMC-2910 suitable for use in the present invention, has a viscosity of about 15000 cps (15 millipascal-seconds) at 20C in a 2% aqueous solution as determined by a Ubbelohde viscometer. As used herein, "degree of substitution" means the average number of substituent groups attached to an anhydroglucose ring, and "hydroxypropyl molar substitution" means the number of moles of hydroxypropyl per mole anhydroglucose.

One suitable polyvinyl alcohol and polyethylene glycol copolymer is commercially available from BASF Corporation under the tradename KOLLICOAT IR.

As used herein, "modified starches" include starches that have been modified by crosslinking, chemically modified for improved stability or optimized performance, or physically modified for improved solubility properties or optimized performance. Examples of chemically-modified starches are well known in the art and typically include those starches that have been chemically treated to cause replacement of some of its hydroxyl groups with either ester or ether groups. Crosslinking, as used herein, may occur in modified starches when two hydroxyl groups on neighboring starch molecules are chemically linked. As used herein, "pre-gelatinized starches" or "instantized starches" refers to modified starches that have been pre-wetted, then dried to enhance their coldwater solubility.

Suitable modified starches are commercially available from several suppliers such as, for example, A. E. Staley Manufacturing Company, and National Starch & Chemical Company. One suitable film forming modified starch includes the pre-gelatinized waxy maize derivative starches that are commercially available from National Starch & Chemical Company under the tradenames PURITY GUM and FILMSET, and derivatives, copolymers, and mixtures thereof. Such waxy maize starches typically contain, based upon the total weight of the starch, from about 0 percent to about 18 percent of amylose and from about 100% to about 88% of amylopectin.

Other suitable film forming modified starches include the hydroxypropylated starches, in which some of the hydroxyl groups of the starch have been etherified with hydroxypropyl groups, usually via treatment with propylene oxide. One example of a suitable hydroxypropyl starch that possesses film-forming properties is available from Grain Processing Company under the tradename, PURE-COTE B790.

Suitable tapioca dextrins for use as film formers include those available from National Starch & Chemical Company under the tradenames CRYSTAL GUM or K-4484, and derivatives thereof such as modified food starch derived from tapioca, which is available from National Starch and Chemical under the tradename PURITY GUM 40, and copolymers and mixtures thereof.

Any thickener known in the art is suitable for use in the flowable material of the present invention. Examples of such thickeners include but are not limited to hydrocolloids (also referred to herein as gelling polymers), clays, gelling starches, and crystallizable carbohydrates, and derivatives, copolymers and mixtures thereof.

Examples of suitable hydrocolloids (also referred to herein as gelling polymers) such as alginates, agar, guar gum, locust bean, carrageenan, tara, gum arabic, tragacanth, pectin, xanthan, gellan, maltodextrin, galactomannan, pusstulan, laminarin, scleroglucan, gum arabic, inulin, pectin, whelan, rhamsan, zooglan, methylan, chitin, cyclodextrin, chitosan. Examples of suitable clays include smectites such as bentonite, kaolin, and laponite; magnesium trisilicate, magnesium aluminum silicate, and the like, and derivatives and mixtures thereof. Examples of suitable gelling starches include acid hydrolyzed starches, and derivatives and mixtures thereof. Additional suitable thickening hydrocolloids include low-moisture polymer solutions such as mixtures of gelatin and other hydrocolloids at water contents up to about 30%, such as for example those used to make "gummi" confection forms.

Additional suitable thickeners include crystallizable carbohydrates, and the like, and derivatives and combinations thereof.

Suitable crystallizable carbohydrates include the monosaccharides and the oligosaccharides. Of the monosaccharides, the aldohexoses e.g., the D and L isomers of allose, altrose, glucose, mannose, gulose, idose, galactose, talose, and the ketohexoses e.g., the D and L isomers of fructose and sorbose along with their hydrogenated analogs: e.g., glucitol (sorbitol), and mannitol are preferred. Of the oligosaccharides, the 1,2-disaccharides sucrose and trehalose, the 1,4-disaccharides maltose, lactose, and cellobiose, and the 1,6-disaccharides gentiobiose and melibiose, as well as the trisaccharide raffinose are preferred along with the isomerized form of sucrose known as isomaltulose and its hydrogenated analog isomalt. Other hydrogenated forms of reducing disaccharides (such as maltose and lactose), for example, maltitol and lactitol are also preferred. Additionally, the hydrogenated forms of the aldopentoses: e.g., D and L ribose, arabinose, xylose, and lyxose and the hydrogenated forms of the aldotetroses: e.g., D and L erythrose and threose are preferred and are exemplified by xylitol and erythritol, respectively.

In one embodiment of the invention, the flowable material comprises gelatin as a gelling polymer. Gelatin is a natural, thermogelling polymer. It is a tasteless and colorless mixture of derived proteins of the albuminous class, which is ordinarily soluble in warm water. Two types of gelatin --Type A and Type B -- are commonly used. Type A gelatin is a derivative of acid-treated raw materials. Type B gelatin is a derivative of alkali-treated raw materials. The moisture content of gelatin, as well as its Bloom strength, composition and original gelatin processing conditions, determine its transition temperature between liquid and solid. Bloom is a standard measure of the strength of a gelatin gel, and is roughly correlated with molecular weight. Bloom is defined as the weight in grams required to move a half-inch diameter plastic plunger 4 mm into a 6.67% gelatin gel that has been held at 10C for 17 hours. In a preferred embodiment, the flowable material is an aqueous solution comprising 20% 275 Bloom pork skin gelatin, 20% 250 Bloom Bone Gelatin, and approximately 60% water.

Suitable xanthan gums include those available from C. P. Kelco Company under the tradenames KELTROL 1000, XANTROL 180, or K9B310.

Suitable clays include smectites such as bentonite, kaolin, and laponite; magnesium trisilicate, magnesium aluminum silicate, and the like, and derivatives and mixtures thereof.

"Acid-hydrolyzed starch," as used herein, is one type of modified starch that results from treating a starch suspension with dilute acid at a temperature below the gelatinization point of the starch. During the acid hydrolysis, the granular form of the starch is maintained in the starch suspension, and the hydrolysis reaction is ended by neutralization, filtration and drying once the desired degree of hydrolysis is reached. As a result, the average molecular size of the starch polymers is reduced. Acid-hydrolyzed starches (also known as "thin boiling starches") tend to have a much lower hot viscosity than the same native starch as well as a strong tendency to gel when cooled.

"Gelling starches," as used herein, include those starches that, when combined with water and heated to a temperature sufficient to form a solution, thereafter form a gel upon cooling to a temperature below the gelation point of the starch. Examples of gelling starches include, but are not limited to, acid hydrolyzed starches such as that available from Grain Processing Corporation under the tradename PURE-SET B950; hydroxypropyl distarch phosphate such as that available from Grain Processing Corporation under the tradename, PURE-GEL B990, and mixtures thereof.

Suitable low-melting hydrophobic materials include fats, fatty acid esters, phospholipids, and waxes. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes include carnauba wax, spermaceti wax, beeswax, candelilla wax, shellac wax, microcrystalline wax, and paraffin wax; fat-containing mixtures such as chocolate; and the like.

Suitable non-crystallizable carbohydrates include non-crystallizable sugars such as polydextrose, and starch hydrolysates, e.g. glucose syrup, corn syrup, and high fructose corn syrup; and non-crystallizable sugar-alcohols such as maltitol syrup.

Suitable solvents for optional use as components of the flowable material for making the core or the shell by molding include water; polar organic solvents such as methanol, ethanol, isopropanol, acetone, and the like; and non-polar organic solvents such as methylene chloride, and the like; and mixtures thereof.

The flowable material for making cores or the shell by molding may optionally comprise adjuvants or excipients, which may comprise up to about 30% by weight of the flowable material. Examples of suitable adjuvants or excipients include plasticizers, detackifiers, humectants, surfactants, anti-foaming agents, colorants, flavorants, sweeteners, opacifiers, and the like.

Suitable plasticizers for making the core, the shell, or a portion thereof, by molding include, but not be limited to polyethylene glycol; propylene glycol; glycerin; sorbitol; triethyl citrate; tribuyl citrate; dibutyl sebecate; vegetable oils such as castor oil, rape oil, olive oil, and sesame oil; surfactants such as polysorbates, sodium lauryl sulfates, and dioctyl-sodium sulfosuccinates; mono acetate of glycerol; diacetate of glycerol; triacetate of glycerol; natural gums; triacetin; acetyltributyl citrate; diethyloxalate; diethylmalate; diethyl fumarate; diethylmalonate; dioctylphthalate; dibutylsuccinate; glyceroltributyrate; hydrogenated castor oil; fatty acids; substituted triglycerides and glycerides; and the like and/or mixtures thereof. In one embodiment, the plasticizer is triethyl citrate. In certain embodiments, the shell is substantially free of plasticizers, i.e. contains less than about 1%, say less than about 0.01% of plasticizers.

In embodiments in which the shell is prepared using a solvent-free molding process, the shell typically comprises at least about 30 percent, e.g. at least about 45 percent by weight of a thermal-reversible carrier. The shell may optionally further comprise up to about 55 weight percent of a release-modifying excipient. The shell may optionally further comprise up to about 30 weight percent total of various plasticizers, adjuvants and excipients. In certain embodiments in which the shell is prepared by solvent-free molding, and functions to delay the release of one or more active ingredients from an underlying core, the release modifying excipient is preferably selected from swellable, crodible hydrophilic materials.

In embodiments in which the shell is prepared using a solvent-based molding process, the shell typically comprises at least about 10 weight percent, e.g. at least about 12 weight percent or at least about 15 weight percent or at least about 20 weight percent or at least about 25 weight percent of a film-former. Here, the shell may optionally further comprise up to about 55 weight percent of a release-modifying excipient. The shell may again also optionally further comprise up to about 30 weight percent total of various plasticizers, adjuvants, and excipients.

The total weight of the shell is preferably about 20 percent to about 400 percent of the total weight of the cores. In embodiments wherein the shell is prepared by a solvent-free molding process, the total weight of the shell is typically from about 50 percent to about 400 percent, e.g. from about 75 percent to about 400 percent, or about 100 percent to about 200 percent of the total weight of the cores. In embodiments wherein the shell is prepared by a solvent-based molding process, the total weight of the shell is typically from about 20 percent to about 100 percent of the total weight of the cores.

The thickness of the shell is important to the release properties of the dosage form. Advantageously, the dosage forms of the invention can be made with precise control over shell thickness, in particular using the thermal cycle or thermal setting injection molding methods and apparatus described above. Typical shell thicknesses that may be employed are about 50 to about 4000 microns. In certain preferred embodiments, the shell has a thickness of less than 800 microns. In embodiments wherein the shell portion is prepared by a solvent-free molding process, the shell portion typically has a thickness of about 500 to about 4000 microns, e.g. about 500 to about 2000 microns, say about 500 to about 800 microns, or about 800 to about 1200 microns. In embodiments wherein the shell portion is prepared by a solvent-based molding process, the shell portion typically has a thickness of less than about 800 microns, e.g. about 100 to about 600 microns, say about 150 to about 400 microns. In a particularly preferred embodiment the dosage form comprises first and second cores and first and second shell portions, and at least one of the shell portions has a thickness of less than about 800 microns, e.g. about 100 to about 600 microns, e.g. about 150 to about 400 microns.

In embodiments in which the shell is prepared by molding, either by a solvent-free process or by a solvent-based process, the shell typically is substantially free of pores in the diameter range of 0.5 to 5.0 microns, i.e. has a pore volume in the pore diameter range of 0.5 to 5.0 microns of less than about 0.02 cc/g, preferably less than about 0.01 cc/g, more preferably less than about 0.005 cc/g.

Typical compressed materials have pore volumes in this diameter range of more than about 0.02 cc/g. Pore volume, pore diameter and density may be determined using a Quantachrome Instruments PoreMaster 60 mercury intrusion porosimeter and associated computer software program known as "Porowin." The procedure is documented in the Quantachrome Instruments PoreMaster Operation Manual. The PoreMaster determines both pore volume and pore diameter of a solid or powder by forced intrusion of a non-wetting liquid (mercury), which involves evacuation of the sample in a sample cell (penetrometer), filling the cell with mercury to surround the sample with mercury, applying pressure to the sample cell by: (i) compressed air (up to 50 psi maximum); and (ii) a hydraulic (oil) pressure generator (up to 60000 psi maximum). Intruded volume is measured by a change in the capacitance as mercury moves from outside the sample into its pores under applied pressure. The corresponding pore size diameter (d) at which the intrusion takes place is calculated directly from the so-called "Washburn Equation" where gamma is the surface tension of liquid mercury, theta is the contact angle between mercury and the sample surface and P is the applied pressure.

In those embodiments in which solvent-free molding is employed, the flowable material may comprise a thermal-reversible carrier.

Suitable thermal-reversible carriers for use in making a core, the shell or both by molding are thermoplastic materials typically having a melting point below about 110C, more preferably between about 20 and about 100C.

Suitable compositions for such applications contain at least about 20% by weight of a thermal reversible carrier, preferably at least about 30% by weight.

Examples of suitable thermal-reversible carriers for solvent-free molding include thermoplastic polyalkylene glycols, thermoplastic polyalkylene oxides, low melting hydrophobic materials, thermoplastic polymers, thermoplastic starches, and the like. Preferred thermal-reversible carriers include polyethylene glycol and polyethylene oxide.

Suitable thermoplastic polyalkylene glycols for use as thermal-reversible carriers include polyethylene glycol having molecular weight from about 100 to about 20,000, e.g. from about 100 to about 8,000 Daltons.

Suitable thermoplastic polyalkylene oxides include polyethylene oxide having a molecular weight from about 100,000 to about 900,000 Daltons.

Suitable low-melting hydrophobic materials for use as thermal-reversible carriers include fats, fatty acid esters, phospholipids, and waxes which are solid at room temperature, fat-containing mixtures such as chocolate; and the like. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes that are solid at room temperature include carnauba wax, spermaceti wax, beeswax, candelilla wax, shellac wax, microcrystalline wax, and paraffin wax.

Suitable thermoplastic polymers for use as thermal-reversible carriers include thermoplastic water swellable cellulose derivatives, thermoplastic water insoluble polymers, thermoplastic vinyl polymers, thermoplastic starches, and thermoplastic resins, and combinations thereof.

Suitable thermoplastic water swellable cellulose derivatives include hydroxypropylmethyl cellulose (HPMC), methyl cellulose (MC), carboxymethylcellulose (CMC), cross-linked hydroxypropylcellulose, hydroxypropyl cellulose (HPC), hydroxybutylcellulose (HBC), hydroxyethylcellulose (HEC), hydroxypropylethylcellulose, hydroxypropylbutylcellulose, hydroxypropylethylcellulose, and salts, derivatives, copolymers, and combinations thereof.

Suitable thermoplastic water insoluble polymers include ethylcellulose, polyvinyl alcohols, polyvinyl acetate, polycaprolactones, cellulose acetate and its derivatives, acrylates, methacrylates, acrylic acid copolymers, and the like and derivatives, copolymers, and combinations thereof.

Suitable thermoplastic vinyl polymers include polyvinylacetate, polyvinyl alcohol, and polyvinyl pyrrolidone (PVP). Examples of suitable thermoplastic starches for use as thermal-reversible carriers are disclosed for example in U.S. Pat. No. 5,427,614. Examples of suitable thermoplastic resins for use as thermal-reversible carriers include dammars, mastic, rosin, shellac, sandarac, and glycerol ester of rosin. In one embodiment, the thermal-reversible carrier for making a core by molding is selected from polyalkylene glycols, polyalkylene oxides, and combinations thereof.

In embodiments in which the shell is made via solvent-free molding, a thermal-reversible carrier is employed in the flowable material to make the shell, said thermal-reversible carrier preferably selected from polyethylene glycol with weight average molecular weight from about 1450 to about 20000, polyethylene oxide with weight average molecular weight from about 100,000 to about 900,000, and the like.

The following non-limiting examples further illustrate the claimed invention.

### Example 1 Preparation of the immediate release ibuprofen core tables

**Table 1: Tablet Blend Formulation:**

| Granulation | Trade Name | Manufacturer | Mg/Tablet |
|---|---|---|---|
| Ibuprofen granules (115 microns) | | Albemarle Corp. Orangeburg, SC | 300.0 |
| Croscarmellose sodium | Ac-Di-Sol | FMC Corp. Philadelphia, PA | 18.65 |
| Glyceryl Behenate, NF | Compritol 888 ATO | Gattefosse Corporation, Westwood. NJ | 18.65 |
| Colloidal silicon dioxide | Cab-O-Sil LM-5^{®} | Cabot Corp. Tuscola, IL | 1.7 |
| Total | | | 339.0 |

### Manufacturing process:

Ibuprofen , glyceryl behenate and croscarmellose sodium were screened through a 30 mesh screen and placed into in a 1 qt. P-K blender for 5 minutes. Colloidal silicon dioxide was added to the blended mixture and mixed for another 5 minutes. A Carver single punch tablet press was equipped with either one set of 0.3925" x 0.4620" double shape tooling (for a dosage form containing two cores) or one set of 0.1623" x 0.3090" ARC FF tooling (for a dosage form containing three cores). The ibuprofen final blend was fed into the cavities mold of the press and was pressed into solid drug cores. The compressed double (two) cores were obtained. The large core weighed 226 mg and contained 200 mg of ibuprofen. The small core weighed 113 mg and contained 100 mg of ibuprofen. The compressed triple (three) cores were obtained. Three separate cores were obtained and each core weighed 113 mg and contained 100 mg of ibuprofen.

### Example 2

Preparation of the molded ibuprofen integrated tablet, containing 300 mg ibuprofen in double cores by aqueous molding process

**Table 2: Shell formulation for first half shell:**

| Ingredient | Trade Name | Manufacturer | Weight (g) |
|---|---|---|---|
| Gelatin, NF | 275 Bloom Type A Porkskin | Kind & Knox Gelatin, Inc., Sioux City, Iowa | 30.0 |
| D. I. Water | | | 70.0 |
| Total | | | 100 |

**Table 3: Shell formulation for second half shell:**

| Ingredient | Trade Name | Manufacturer | Weight (g) |
|---|---|---|---|
| Gelatin, NF | 275 Bloom Type A Porkskin | Kind & Knox Gelatin, Inc., Sioux City, Iowa | 30.0 |
| D. I. Water | | | 69.99 |
| D&C Red Dye # 33 | | Colorcon, West Point, PA | 0.01 |
| Total | | | 100 |

**Table 4: Shell weight gain for molded ibuprofen integrated tablet containing ibuprofen double cores**

| Ingredient | Mg/Dosage Form | % W/W |
|---|---|---|
| Gelatin Coating | 157.0 | 31.5 |
| Ibuprofen Double (2) Cores | 341.0 | 68.5 |
| Total | 498.0 | 100.0 |

### Manufacturing process:

The shell materials were prepared by adding either gelatin (first shell formulation) or gelatin and color dye (second shell formulation) into glass bottles. D.I. water were added to the bottles and mixed with a spatula. The bottles were scaled with a cap. The bottles were kept at 55°C forced-air oven overnight. The shell materials were provided in flowable form.

A laboratory scale thermal cycle molding unit was used to apply the first and second shell portions to the cores, and comprised a single mold assembly made from an upper mold assembly portion comprising an upper mold cavity, and a lower mold assembly portion comprising a lower mold cavity. The lower mold assembly portion was first cycled to a hot stage at 70°C for 60 seconds. The first shell material from Table 2 was introduced into the lower mold cavity. The ibuprofen double cores (from Example 1) were then inserted into a blank upper mold assembly. The blank upper mold assembly used for the double cores had a dividing wall between the housing units to hold two cores. The blank upper mold assembly portion was mated the lower mold assembly portion. The mold assembly was then cycled to a cold stage at 10°C for 25 seconds to harden the first shell portion. The blank mold assembly portion was removed from the lower mold assembly portion. The upper mold assembly portion was cycled to a hot stage at 70°C for 30 seconds. The second shell material from Table 3 was added to the upper mold cavity. The lower mold assembly portion, which was maintained at 10°C, was then mated with the upper mold assembly portion. Both the upper and lower mold assembly portions were cycled to a cold stage at 10°C for 120 seconds to harden the second shell portion. The lower mold assembly portion was then removed and the finished dosage form, a molded coated integrated tablet with two halves of the same shell material, was ejected from the upper mold cavity. The finished dosage form was dried at room temperature for 12 hours to remove all residual water. The weight gain due to the shell material (i.e. the difference in weight between the finished dosage form, and the cores) was recorded. When light shined through the finished dosage form, a red effect was seen through the dividing portions of the top and, bottom faces and one of the side faces.

### Example 3

Preparation of the molded ibuprofen integrated tablet, containing 300 mg of ibuprofen in triple cores by aqueous molding process

**Table 5: Shell weight gain for molded ibuprofen integrated tablet containing ibuprofen triple (3) cores**

| Ingredient | Mg/Dosage Form | % W/W |
|---|---|---|
| Gelatin Coating | 139.0 | 29.3 |
| Ibuprofen Triple (3) Cores | 335.0 | 70.7 |
| Total | 474.0 | 100.0 |

### Manufacturing process:

A laboratory scale thermal cycle molding unit applied the first and second shell portions to the cores, and comprised a single mold assembly made from an upper mold assembly portion comprising an upper mold cavity, and a lower mold assembly portion comprising a lower mold cavity. The lower mold assembly portion was first cycled to a hot stage at 70°C for 60 seconds. The first shell material (from Table 2) was introduced into the lower mold cavity. The ibuprofen triple cores (from Example 1) were then inserted into a blank upper mold assembly. The blank upper mold assembly used for the triple cores had three dividing walls among the housing units to hold three cores. The blank upper mold assembly portion was mated the lower mold assembly portion. The mold assembly was then cycled to a cold stage at 10°C for 25 seconds to harden the first shell portion. The blank mold assembly portion was removed from the lower mold assembly portion. The upper mold assembly portion was cycled to a hot stage at 70°C for 30 seconds. The second shell material (from Table 3) was added to the upper mold cavity. The lower mold assembly portion, which was maintained at 10°C, was then mated with the upper mold assembly portion. Both the upper and lower mold assembly portions were cycled to a cold stage at 10°C for 120 seconds to harden the second shell portion. The lower mold assembly portion was then removed and the finished dosage form, a molded coated integrated tablet with two halves of the same shell material, was ejected from the upper mold cavity. The finished dosage form was dried at room temperature for 12 hours to remove all residual water. The weight gain due to the shell material (i.e. the difference in weight between the finished dosage form, and the cores) was recorded. When light shined through the finished dosage form, a red effect could be seen through the dividing portions of the top and bottom faces of the dosage form, but not through the side faces.

### Example 4

### Preparation of the molded ibuprofen tablet, containing 300 mg of ibuprofen in double cores by solvent free molding process

**Table 6: Shell formulation for first half shell:**

| Ingredient | Trade Name | Manufacturer | Weight (g) |
|---|---|---|---|
| Polyethylene Glycol 3350 | Carbowax^{®} 3350 | Union Carbide Corporation, Danbury, CT | 27.0 |
| Polyethylene Glycol 8000 | Carbowax^{®} 8000 | Union Carbide Corporation, Danbury, CT | 40.5 |
| Poly (ethylene oxide), 100,000 | Polyox^{®} N-10 | Union Carbide Corporation, Danbury, CT | 7.5 |
| Lauroyl Macrogul-32 Glycerides | Gelucire^{®} 50/13 | Gattefosse Corporation, Westwood. NJ | 10.0 |
| Propylene Glycol | | Union Carbide Corporation, Danbury, CT | 15.0 |
| Total | | | 100.0 |

**Table 7: Shell formulation for second half shell:**

| Ingredient | Trade Name | Manufacturer | Weight (g) |
|---|---|---|---|
| Polyethylene Glycol 3350 | Carbowax^{®} 3350 | Union Carbide Corporation, Danbury, CT | 27.0 |
| Polyethylene Glycol 8000 | Carbowax^{®} 9000 | Union Carbide Corporation, Danbury, CT | 40.5 |
| Poly (ethylene oxide), 100,000 | Polyox ^{®} N-10 | Union Carbide Corporation, Danbury, CT | 7.5 |
| Lauroyl Macrogol-32 Glycerides | Gelucire^{®} 50/13 | Gattefosse Corporation, Westwood. NJ | 10.0 |
| Propylene Glycol | | Union Carbide Corporation, Danbury, CT | 14.9 |
| D&C Yellow # 10 | | Colorcon, West Point, PA | 0.1 |
| Total | | | 100.0 |

**Table 8: Shell weight gain for molded ibuprofen integrated tablet containing double (2) cores**

| Ingredient | Mg/Dosage Form | % W/W |
|---|---|---|
| Solvent Free Shell Coating | 500.0 | 59.6 |
| Ibuprofen Double Cores | 339.0 | 40.4 |
| Total | 839.0 | 100.0 |

### Manufacturing process:

The shell material was prepared by first submersing a beaker in a 90°C water bath (Ret digi-visc; Antal-Direct, Wayne, PA). Polyethylene glycol 3350, polyethylene glycol 8000, polyethylene oxide 100,000 and lauroyl macrogol-32 glycerides were added to the beaker and were mixed with a mixer until all powders were melted. Either propylene glycol (first shell formulation) or propylene glycol and color dye (second shell formulation) was added and was mixed for 60 minutes. The shell material was provided in flowable form.

A laboratory scale thermal cycle molding unit applied the first and second shell portions to the cores, and was comprised of a single mold assembly made from an upper mold assembly portion comprising an upper mold cavity, and a lower mold assembly portion comprising a lower mold cavity. The lower mold assembly portion was first cycled to a hot stage at 90°C for 60 seconds. The first shell material from Table 6 was introduced into the lower mold cavity. The double cores (from Example 1) were then inserted into a blank upper mold assembly. The blank upper mold assembly used for the double cores had a dividing wall between the housing units to hold two cores. The blank upper mold assembly portion was mated the lower mold assembly portion. The mold assembly was then cycled to a cold stage at 5°C for 30 seconds to harden the first shell portion. The blank mold assembly portion was removed from the lower mold assembly portion. The upper mold assembly portion was cycled to a hot stage at 90°C for 30 seconds. The second shell material from Table 7 was added to the upper mold cavity. The lower mold assembly portion, which was maintained at 5°C, was then mated with the upper mold assembly portion. Both the upper and lower mold assembly portions were cycled to a hot stage at 90°C for 10 seconds and then were cycled to a cold stage at 5°C for 120 seconds to harden the second shell portion. The lower mold assembly portion was then removed and the finished dosage form, a molded coated integrated tablet with two halves of the same shell material, was ejected from the upper mold cavity. The weight gain due to the shell material (i.e. the difference in weight between the finished dosage form, and the cores) was recorded. When light shined through the finished dosage form, a somewhat opaque yellow effect could be seen through the dividing portions of the top and bottom faces and one of the side faces.

### Example 5

### Preparation of the molded ibuprofen tablet, containing 300 mg of ibuprofen in triple (3) cores by solvent free molding process

**Table 9: Shell weight gain for molded ibuprofen integrated tablet containing triple cores**

| Ingredient | Mg/Dosage Form | % W/W |
|---|---|---|
| Solvent Free Shell Coating | 522.0 | 62.1 |
| Ibuprofen Triple (3) Cores | 318.0 | 37.9 |
| Total | 840.0 | 100.0 |

### Manufacturing process:

A laboratory scale thermal cycle molding unit applied the first and second shell portions to the cores, and was comprised of a single mold assembly made from an upper mold assembly portion comprising an upper mold cavity, and a lower mold assembly portion comprising a lower mold cavity. The lower mold assembly portion was first cycled to a hot stage at 90°C for 60 seconds. The first shell material (from Table 6) was introduced into the lower mold cavity. The triple cores (from Example 1) were then inserted into a blank upper mold assembly. The blank upper mold assembly used for the triple cores had three dividing walls among the housing units to hold three cores. The blank upper mold assembly portion was mated the lower mold assembly portion. The mold assembly was then cycled to a cold stage at 5°C for 30 seconds to harden the first shell portion. The blank mold assembly portion was removed from the lower mold assembly portion. The upper mold assembly portion was cycled to a hot stage at 90°C for 30 seconds. The second shell material (from Table 7) was added to the upper mold cavity. The lower mold assembly portion, which was maintained at 5°C, was then mated with the upper mold assembly portion. Both the upper and lower mold assembly portions were cycled to a hot stage at 90°C for 10 seconds and then were cycled to a cold stage at 5°C for 120 seconds to harden the second shell portion. The lower mold assembly portion was then removed and the finished dosage form, a molded coated integrated tablet with two halves of the same shell material, was ejected from the upper mold cavity. The weight gain due to the shell material (i.e. the difference in weight between the finished dosage form, and the cores) was recorded. When light shined through the finished dosage form, a somewhat yellow opaque effect could be seen through the dividing portions of the top and bottom faces of the dosage form, but not through the side face.

### Example 6: Analysis of Light Transmission

### Equipment used for light transmission measurements:

1. Fiber Optic Illuminator (Fiber-Lite Bausch & Lomb)
2. Light Meter (Fischer Scientific S/N 61800692 06-662-64)
3. Two stainless steel blocks (Width: 2.5 cm; Height: 12.8 cm; Length: 10 cm)
4. Ring Stand with clamp
5. Filter paper (Scientific Products weighing paper 4x4 inch)
6. Card board (10x10 cm)
7. Ring (Height: 2 cm; Inner Diameter: 3.2 cm; Outer Diameter: 5.1cm)
8. Coated Tablet Sample with multiple core potions and light transmitting layers

### Set up procedure of the light transmission apparatus:

The steel blocks are set parallel to one another. The distance between the two steel blocks is 5 cm apart. A 10 x 10 cm square block of cardboard is prepared. Tape is placed around the edges of the blocks to secure their position. Filter paper is then placed on the blocks and taped to the sides to secure, and is used to diffuse the intensity of the light source. The cardboard piece is measured for length and width and the center is marked. In the center of the cardboard a whole is cut out to match the size of the tablet sample, based on the width of the flat faced sample. The cardboard is then positioned onto the filter paper so that the edges are aligned and then the cardboard is secured with tape on all sides. The 10 x 10 cm platform (including the steel block, the cardboard and the filter paper) is secured. The total height of the platform is approximate 13 cm.

The separating ring is centered on the top of the platform around the opening in the cardboard, and is used to separate an approximate 2-cm distance between the light meter and the sample. The ring is also used as a placement for the light meter. The position of the ring is marked onto the cardboard by evenly measuring the width and length from the opening. The fiber optic illuminator is placed to the side of the platform. An optic fiber light is adjusted in between the steel blocks so that the light from the fiber optic light is shined under the opening in the cardboard. The light is set at a 90-degree angle and is perpendicular to the top of the platform. The fiber optic light is secured by clamping around the light to a ring stand to hold the position.

### Procedure for light transmission measurement:

The light meter is turned on with the cap covering the meter. The zero key in the light meter is pressed. The reading is recorded and is indicated as 0% light transmittance. The illuminator is turned on using the lowest intensity setting. The ring is placed on the marked areas and is secured. The light meter is placed over the ring.

The meter is adjusted until a stable reading is obtained. The reading of the light transmittance is recorded as 100% light transmittance. The meter is removed. The sample is placed into the opening in the platform so it is submerged in the cardboard. The meter is placed on again over the ring and is adjusted to get a stable reading. The reading is recorded.

### Analysis Performed

Three individual coated tablet samples per example are tested using the above procedure. Coated tablet examples 2, 3, 4 and 5 (prepared above) are analyzed. The data for individual sample and average light transmitted is reported in Tables 10, 11, 12, and 13.

**Table 10: Example 2 Light transmission measurement results:**

| **Example 2** | **Readings (Lux)*** | **Light Transmitted** |
|---|---|---|
| **Control** | 22400 | 100% |
| **Sample-1** | 20 | 0.09% |
| **Sample-2** | 27 | 0.12% |
| **Sample-3** | 16 | 0.07% |
| **Average** | 21 | 0.094% |
| **S.D.** | ±5.6 | ± 0.025 |

| | | |
|---|---|---|
| * Light Measurement is reported in Lux; wherein Lux equals one lumen incident per square meter of illuminated light. | | |

**Table 11: Example 3 Light Transmission measurement results**

| **Example 3** | **Readings (Lux)** | **Light Transmitted** |
|---|---|---|
| **Control** | 22300 | 100% |
| **Sample** 1 | 243 | 1.09% |
| **Sample 2** | 18 | 1.09% |
| **Sample 3** | 13 | 0.06% |
| **Average** | 91.3 | 0.41 % |
| **S.D.** | 131.3 | 0.588 |

**Table 12: Example 4 Light Transmission measurement results**

| **Example 4** | **Readings (Lux)** | **Light Transmitted** |
|---|---|---|
| **Control** | 22400 | 100% |
| **Sample 1** | 1280 | 5.71% |
| **Sample 2** | 1135 | 5.07% |
| **Sample 3** | 1132 | 5.05% |
| **Average** | 1182.3 | 5.28% |
| **SD** | 84.59 | 0.375 |

**Table 13: Example 5 Light Transmission measurement result**

| **Example 5** | **Readings (Lux)** | **Light Transmitted** |
|---|---|---|
| **Control** | 22500 | 100% |
| **Sample 1** | 131 | 5.82% |
| **Sample 2** | 1270 | 5.64% |
| **Sample 3** | 1413 | 6.28% |
| **Average** | 1331 | 5.91% |
| **SD** | 73.7 | 0.33 |

## Claims

1. A solid dosage form comprising at least two separate and distinct compressed portions having at least one surface area, a horizontal and a vertical axis and at least one light transmitting coating that is provided between said at least two separate and distinct compressed portions and covering at least one surface of each of said at least two separate and distinct compressed portions, wherein the light transmitting coating is translucent along at least one axis of the at last two separate and distinct compressed portions, and wherein the light transmitting coating comprises gelatin and a water-soluble dye.

2. A solid dosage term according to claim 1 wherein light can be transmitted through the light transmitting coating along both the horizontal and vertical axis of the pressed portions.

3. The solid dosage form according to claim wherein the light transmitting coating comprises at least 20% by weight of a thermal reversible polymer.

4. The solid dosage form according to claim 3 wherein the light transmitting coating further comprises a colorant.

5. The solid dosage form according to claim 4 wherein the colorant is a dye or a pigment.

6. The solid dosage form according to claim 1 wherein the compressed portions are compressed cores.

7. The solid dosage form according to claim 1 herein at least one pressed comportion is a molded core.

8. The solid dosage form according to claim 1 wherein at least one compressed portion is a compressed core.

9. The solid dosage form according to claim 7 wherein at least one compressed core is a compressed core.

10. The solid dosage form according to claim 1 comprising at least three compressed portions.

11. The solid dosage term according to claim 1 wherein the dosage form allows at least 1% of a full-spectrum beam of viable light to he transmitted along a major axis through the light transmitting coaling.

12. The solid dosage form according to claim 1 wherein the light transmitting light layer is transparent.

13. The solid dosage form according to claim 1 further comprising a colorant that is only visible when subjected to light having a specific wavelength.

14. The solid dosage form according to claim 1 further comprising reflective particles or flakes capable of diffracting light passing through said light transmitting layer.

15. The solid dosage form according to claim 1 further comprising a sensate within the light transmitting layer.

16. The solid dosage form according to claim 1, wherein said at least two compressed portions comprise two compressed cores, wherein each of said two compressed cores has at least one relatively flat face, and wherein a first compressed core hae a first light transmitting layer having a first color and a second compressed core has a second light transmitting layer provided of a different color, wherein said light transmitting layers are provided in contact with each of the relatively flat faces for each compressed core, and wherein each of the light transmitting layers is translucent along at least one axis of the compressed portions.

## Patentansprüche

1. Feste Dosierform, die wenigstens zwei getrennte und verschiedene verpresste Abschnitte mit wenigstens einer Oberfläche, einer horizontalen und einer vertikalen Achse und wenigstens eine lichtdurchlässige Beschichtung umfasst, die zwischen den wenigstens zwei getrennten und verschiedenen verpressten Abschnitten vorgesehen ist und wenigstens eine Oberfläche von jedem der wenigstens zwei getrennten und verschiedenen verpressten Abschnitte überzieht, wobei die lichtdurchlässige Beschichtung entlang wenigstens einer Achse der wenigstens zwei getrennten und verschiedenen verpressten Abschnitte durchscheinend ist und wobei die lichtdurchlässige Beschichtung Gelatine und einen wasserlöslichen Farbstoff umfasst.

2. Feste Dosierform nach Anspruch 1, wobei Licht durch die lichtdurchlässige Beschichtung entlang sowohl der horizontalen als auch vertikalen Achse der verpressten Abschnitte hindurchgelassen werden kann.

3. Feste Dosierform nach Anspruch 1, wobei die lichtdurchlässige Beschichtung wenigstens 20 Gew.-% eines thermisch reversiblen Polymers umfasst.

4. Feste Dosierform nach Anspruch 3, wobei die lichtdurchlässige Beschichtung weiter ein Färbemittel umfasst.

5. Feste Dosierform nach Anspruch 4, wobei das Färbemittel ein Farbstoff oder ein Pigment ist.

6. Feste Dosierform nach Anspruch 1, wobei die verpressten Abschnitte verpresste Kerne sind.

7. Feste Dosierform nach Anspruch 1, wobei wenigstens ein verpresster Abschnitt ein ausgeformter Kern ist.

8. Feste Dosierform nach Anspruch 1, wobei wenigstens ein verpresster Abschnitt ein verpresster Kern ist.

9. Feste Dosierform nach Anspruch 7, wobei wenigstens ein verpresster Kern ein verpresster Kern ist.

10. Feste Dosierform nach Anspruch 1, die wenigstens drei verpresste Abschnitte umfasst.

11. Feste Dosierform nach Anspruch 1, wobei die Dosierform ermöglicht, dass wenigstens 1% eines Vollspektrumstrahls von sichtbarem Licht entlang einer Hauptachse durch die lichtdurchlässige Beschichtung hindurchgelassen wird.

12. Feste Dosierform nach Anspruch 1, wobei die lichtdurchlässige Lichtschicht durchsichtig ist.

13. Feste Dosierform nach Anspruch 1, die weiter ein Färbemittel umfasst, das nur sichtbar ist, wenn es Licht mit einer spezifischen Wellenlänge unterworfen wird.

14. Feste Dosierform nach Anspruch 1, die weiter reflektierende Teilchen oder Schuppen umfasst, die Licht streuen können, das durch die lichtdurchlässige Schicht hindurchgeht.

15. Feste Dosierform nach Anspruch 1, die weiter ein Sensat innerhalb der lichtdurchlässigen Schicht umfasst.

16. Feste Dosierform nach Anspruch 1, wobei die wenigstens zwei verpressten Abschnitte zwei verpresste Kerne umfassen, jeder der zwei verpressten Kerne wenigstens eine relativ flache Fläche aufweist und ein erster verpresster Kern eine erste lichtdurchlässige Schicht mit einer ersten Färbung aufweist und ein zweiter verpresster Kern eine zweite lichtdurchlässige Schicht, die mit einer unterschiedlichen Färbung versehen ist, aufweist, wobei die lichtdurchlässigen Schichten in Kontakt mit jeder der relativ flachen Flächen für jeden verpressten Kern vorgesehen sind und wobei jede der lichtdurchlässigen Schichten entlang wenigstens einer Achse der verpressten Abschnitte durchscheinend ist.

## Revendications

1. Forme de dosage solide comprenant au moins deux parties comprimées séparées et distinctes ayant au moins une surface, un axe horizontal et un axe vertical et au moins un revêtement de transmission de lumière qui est prévu entre lesdites au moins deux parties comprimées séparées et distinctes et recouvrant au moins une surface de chacune desdites au moins deux parties comprimées séparées et distinctes, dans laquelle le revêtement de transmission de lumière est translucide le long d'au moins un axe des au moins deux parties comprimées séparées et distinctes, et dans laquelle le revêtement de transmission de lumière comprend de la gélatine et une teinture soluble dans l'eau.

2. Forme de dosage solide selon la revendication 1, dans laquelle la lumière peut être transmise par le biais du revêtement de transmission de lumière le long des axes horizontal et vertical des parties comprimées.

3. Forme de dosage solide selon la revendication 1, dans laquelle le revêtement de transmission de lumière comprend au moins 20 % en poids d'un polymère thermique réversible.

4. Forme de dosage solide selon la revendication 3, dans laquelle le revêtement de transmission de lumière comprend en outre un colorant.

5. Forme de dosage solide selon la revendication 4, dans laquelle le colorant est une teinture ou un pigment.

6. Forme de dosage solide selon la revendication 1, dans laquelle les parties comprimées sont des noyaux comprimés.

7. Forme de dosage solide selon la revendication 1, dans laquelle au moins une partie comprimée est un noyau moulé.

8. Forme de dosage solide selon la revendication 1, dans laquelle au moins une partie comprimée est un noyau comprimé.

9. Forme de dosage solide selon la revendication 7, dans laquelle au moins un noyau comprimé est un noyau comprimé.

10. Forme de dosage solide selon la revendication 1, comprenant au moins trois parties comprimées.

11. Forme de dosage solide selon la revendication 1, dans laquelle la forme de dosage permet de transmettre au moins 1 % d'un faisceau de spectre complet de lumière visible le long d'un axe majeur par le biais du revêtement de transmission de lumière.

12. Forme de dosage solide selon la revendication 1, dans laquelle la couche de transmission de lumière est transparente.

13. Forme de dosage solide selon la revendication 1, comprenant en outre un colorant qui n'est visible que lorsqu'il est soumis à la lumière ayant une longueur d'onde spécifique.

14. Forme de dosage solide selon la revendication 1, comprenant en outre des particules réfléchissantes ou paillettes pouvant diffracter la lumière passant par ladite couche de transmission de lumière.

15. Forme de dosage solide selon la revendication 1, comprenant en outre un capteur à l'intérieur de la couche de transmission de lumière.

16. Forme de dosage solide selon la revendication 1, dans laquelle lesdites au moins deux parties comprimées comprennent deux noyaux comprimés, dans laquelle chacun desdits deux noyaux comprimés a au moins une face relativement plate et dans laquelle un premier noyau comprimé a une première couche de transmission de lumière ayant une première couche et un second noyau comprimé a une seconde couche de transmission de lumière prévue avec une couleur différente, dans laquelle lesdites couches de transmission de lumière sont prévues en contact avec chacune des faces relativement plates pour chaque noyau comprimé, et dans laquelle chacune des couches de transmission de lumière est translucide le long d'au moins un axe des parties comprimées.
